# EUROPEAN PATENT APPLICATION

(11) **EP 4 316 366 A1**
(43) Date of publication of application: **07.02.2024**
(21) Application number: 22189145.0
(22) Date of filing: 05.08.2022
(51) Int. Cl.: A61B 5/08, A61B 5/097, A61B 5/00

(54) **PROTECTIVE GEAR WITH INTEGRAL TESTING COMPONENT AND METHOD OF USING THE PROTECTIVE GEAR TO DETERMINE PRESENCE OF INFECTIOUS AGENT**

(71) Applicant: Ares Trading S.A., 1170 Aubonne (CH)
(72) Inventor: VASSALLO, Oscar, 1170 Aubonne (CH)
(74) Representative: Merck Serono S.A. Intellectual Property

(57) **Abstract**

Described herein is protective gear to protect against infectious agents and an analytical method for determining the presence of an infectious agent. The analytical method uses sample collected from air, in particular exhaled breath, to determine the presence of an infectious agent in such air or breath sample.

## Description

### Field of the Invention.

The invention is directed to a protective gear to protect against infectious agents and an analytical method of determining the presence of an infectious agent.

### Background of the Invention.

The COVID-19 pandemic, also known as the coronavirus pandemic, is an ongoing pandemic of coronavirus disease 2019 (COVID-19) caused by severe acute respiratory syndrome coronavirus 2 (SARS-CoV-2). It was first identified in December 2019 in Wuhan, China. The World Health Organization declared the outbreak a Public Health Emergency of International Concern in January 2020 and a pandemic in March 2020. As of July 2022, more than 547 million cases have been confirmed, with more than 6.34 million deaths attributed to COVID-19 with an average of 880000 new cases per day.

Among the diagnostic tests, an increasing need for Covid19 rapid antigen test has spread globally. One of the main problems, as for almost all the Covid19 rapid test, is the invasive and uncomfortable nasopharyngeal swab sample collection step, and the pain due to several recurring tests. A lot of individuals, among health workers and workers subjected to several testing procedures per week, report an increasing discomfort and for this reason, they are starting to refuse to take the test with severe consequences for public health and pandemic dispersion.

Another specific problem related to this nasopharyngeal swab sample collection is needed to have skilled specialized personnel to perform the test and the total test time.

The nasopharyngeal swab sample collection step shows moreover the well-known specimen concentration issue due to the intrinsically low maximum collectable antigen amount. Weakly positive subjects in fact are often difficult to recognize. Also, false-positive tests represent one of the biggest issues related to the market available kits.

### Summary of the Invention.

Covid 19 Antigens and other similar antigens are generally detectable in upper respiratory specimens during the acute phase of infection. A breath emission rate of at least hundred thousand Covid 19 virions per hour is estimated to be present in positive subjects. This specimen concentration is perfectly inside a typical ELISA test useful concentration range.

As such, and as a solution to the above described shortcomings in determining the presence of an infectious agent in a subject suspecting such infection in respect to ease of use and accuracy, in the current invention is directed to an Antigen Rapid Test coupled with a modified protective mask in order to have a new Antigen Rapid Test Mask and a method to determine the presence of an infectious agent using such integral protective mask. In an embodiment of the invention is to have a Lateral Flow Immunoassay Test directly performed on the mask with a Minimum Total Test time of 30 minutes or less.

In a further embodiment is to put the sample well into the inner side of the mask, then breathe for a period of at least 15 minutes for the Exhaled breath Condensate collection, optionally including a final sneeze or cough into the mask.

Accordingly, in one embodiment of the present invention there is provided a rapid test method for determining exposure of a mammal to an infectious agent, the method comprising;
a. collecting a sample from the mammal using a wearable device comprising a sample collection element, and
b. determining the presence of the infectious agent or infectious agent materials in the sample collection element,
wherein the sample collection element is collecting a sample from exhaled breath of the mammal and wherein the presence of the infectious agent or infections agent materials in the sample collection element indicates exposure of the mammal to the infectious agent.

In another embodiment of the present invention there is provided a protective gear against an infectious agent comprising a sample collection element. Preferably the sample collection element comprises a testing element which comprises a rapid antigen test for the infectious agent.

### Brief description of the Drawings.

The following is a brief description of the drawings:

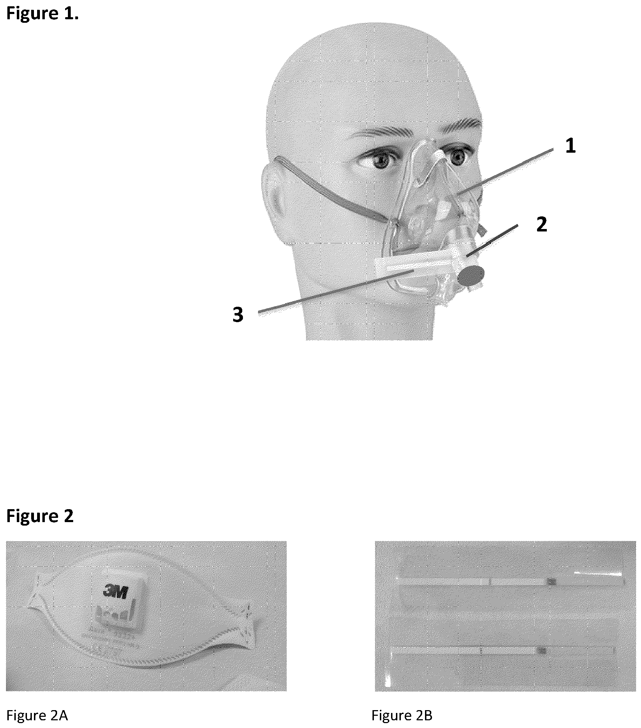

Figure 1: shows a representation of a protective gear in the form of a mask with thereto a collection element which includes a testing element.
Figure 2: shows a representation of a protective mask with a collection element (figure 2A) and a separate testing element (figure 2B).

### Detailed description of the Invention.

Covid 19 Antigens and other similar antigens are generally detectable in upper respiratory specimens during the acute phase of infection. A breath emission rate of at least hundred thousand Covid 19 virions per hour is estimated to be present in positive subjects. This specimen concentration is perfectly inside a typical ELISA test useful concentration range.

As such, and as a solution to the above-described shortcomings in determining the presence of an infectious agent in a subject suspecting such infection in respect to ease of use and accuracy, in the current invention is directed to an Antigen Rapid Test coupled with a modified protective mask in order to have a new Antigen Rapid Test Mask and a method to determine the presence of an infectious agent using such integral protective mask. In an embodiment of the invention is to have a Lateral Flow Immunoassay Test directly performed on the mask with a Minimum Total Test time of 30 minutes or less.

In one such embodiment a sample well is placed into the inner side of the mask, then the subject breathes for 15 minutes or more for the Exhaled breath Condensate collection, optionally including a final sneeze or cough into the mask. In a subsequent step the inert strip is removed allowing the lateral flow immunoassay for another 15 minutes or more to migrate with the test results clearly visible on the window present on the mask surface with a total test time of 30 minutes or more.

Furthermore, the mask inner side may alternatively include a hydro-repellent surface or absorbent gradient surface so as to have a mask exhaled breath condensate concentration in the area of the sample collection element within the mask.

Alternatively, in order to obtain a higher concentration is the breathing time increasing specimen concentration. Such increased sample breathing time could be for a period of 30 or 60 minutes.

The sample collection time is based on an estimated breath emission rate of at least hundred thousand Covid19 virions or other such infections agents per hour. For example, such breath emission rate was estimated from exhaled breath condensate samples from coronavirus symptomatic patients.

For Covid 19 an estimation of ten thousand Covid19 virions possibly exhaled in an hour is assumed. The spike protein trimers, such as those that are targeted in the rapid antigen test, there are known to be 100 copies of spike trimers per virion. As such an estimated 2,5 million of trimers are theoretically exhaled in about 15 min in Covid19 positive subjects with a specimen concentration that is well within a typical ELISA useful concentration range.

Following the existing Lateral flow immunoassay rapid test principle, the Covid19 virions in specimen could be collected in the specimen well at the inner side of the mask after a definite breathing time.

Accordingly, a test to determine the presence of an infectious agent such as Covid 19 using a protective gear in combination with a lateral flow immuno assay can be carried out in about 15 minutes, is accurate and is easy to use by the test subject.

As such, in one embodiment there is provided a rapid test method for determining exposure of a mammal to an infectious agent, the method comprising;
a. collecting a sample from the mammal using a wearable device comprising a sample collection element, and
b. determining the presence of the infectious agent or infectious agent materials in the sample collection element,
wherein the sample collection element is collecting a sample from exhaled breath of the mammal and wherein the presence of the infectious agent or infections agent materials in the sample collection element indicates exposure of the mammal to the infectious agent.

In the method of the present invention, the collection sample element is present on the protective gear and includes a testing element, such as for example a laminar flow rapid antigen test. The results of the test are immediately visual on the protective gear with the integrated test method. Alternatively, the sample collection element is removed from the wearable device (protective gear) prior to an analytical assay to determine the presence of the infectious agent.

As indicated the analytical assay includes any rapid assay which would allow for the detection of the presence of the infectious agent in the sample. Preferably, the analytical assay is a rapid antigen test such as a lateral flow assay using a molecule recognizing one or more antigens present on the infectious agent. Such molecules can be any antibody, partial antibody, fusion protein including an antigen recognition moiety, single chain antibody but also include any other protein or peptide having a moiety that recognizes the antigen. Included in such molecules recognizing one or more antigens of the infectious agent are aptamers. Recognizes in the context of the present invention includes the specific binding or interaction between such molecule and the antigen.

The method of the present invention is useful for the detection of any infectious agent, preferably the infectious agent is an airborne infectious agent. Preferably such airborne infectious agent as in the present invention is a corona virus, for example SARS CoV-2 (Covid 19), or an influenza virus

The sample collection element in the method of the present invention is integral in a wearable device, preferably a protective gear. Such protective gear includes for example a face mask (such as a surgical mask or a FFP2 mask) or a face shield.

Further the sample collection element preferably comprises a testing element. In one embodiment of the present invention such testing element comprises a rapid antigen test for the infectious agent. There are a number of rapid antigen test that can be used in the present method and the selection of it depends on the infectious agent whose presence is to be determined or the specificity of the test or the level of detection or accuracy required. In one embodiment the rapid antigen test is selected from a lateral flow immuno- assay, an aptamer-based test, and a combination thereof.

The method of the present invention includes the use of a wearble device by a mammal, preferably a human. As indicated, the wearable device (protective gear) would need to be worn by the mammal for a predetermined amount of time in order to collect a sufficient amount of sample or in a sufficient concentration to be detectable by the analytical testing method. Accordingly, the predetermined amount of time is dependent on the analytical method and the level of detection obtainable by for example in the rapid antigen laminar flow test. The predetermined amount of time is at least 15 min, preferably 15 minutes to a day, preferably 15 min to 4 hours, preferably 15 min to 2 hours, preferably 15 min to 1 hour, preferably 15 min to 45 min. Alternatively, the predetermined period of time is at least 30 min, preferably 30 min to a day, preferably 30 min to 4 hours, preferably 30 min to 2 hours, preferably 30 min to 1 hour, preferably 30 min to 45 min. Alternatively, the predetermined period of time is at least 45 min, preferably 45 min to a day, preferably 45 min to 4 hours, preferably 45 min to 2 hours, preferably 45 min to 1 hour.

In another embodiment the present invention is directed to a protective gear (1) against an infectious agent comprising a sample collection element (2). Such protective gear includes for example a face mask (such as a surgical mask or a FFP2 mask) or a face shield. Preferably, the protective gear integrates with the sample collection element a testing element (3). Such testing element (3) comprises for example a laminar flow rapid antigen test. The results of the test are immediately visual on the protective gear with the integrated test method. Alternatively, the sample collection element is removable from the protective gear prior to an analytical assay to determine the presence of the infectious agent in a separate analytical method, which may be the same analytical assay as integrated in the protective gear.

As indicated the analytical assay, integral in the sample collection element, includes any rapid assay which would allow for the detection of the presence of the infectious agent in the sample. Preferably, the analytical assay is a rapid antigen test such as a lateral flow assay using a molecule recognizing one or more antigens present on the infectious agent. Such molecules can be any antibody, partial antibody, fusion protein including an antigen recognition moiety, single chain antibody but also include any other protein or peptide having a moiety that recognizes the antigen. Included in such molecules recognizing one or more antigens of the infectious agent are aptamers. Recognizes in the context of the present invention includes the specific binding or interaction between such molecule and the antigen.

The method of the present invention is useful for the detection of any infectious agent, preferably the infectious agent is an airborne infectious agent. Preferably such airborne infectious agent as in the present invention is a corona virus, for example SARS CoV-2 (Covid 19), or an influenza virus.

While not intended to be limiting to the scope of the invention the following example is illustrative for the present invention.

### Example 1: A rapid antigen self-test (by lateral flow immunoassay technology solution) directly performed on a FFP2 face mask.

The experimental testing was repeated in one set using the definite FFP2 membrane surfaces, and in a second set spotting the antigen concentrations directly on the sample collection pad of the LFA test. A parallel series of experiment was performed using:
1) Millipore laboratory made tests [Antibody pair: Anti-SARS-CoV-2 Nucleocapsid Antibody]
   Buffer: for the initial testing phosphate buffered saline (PBS) will be used. Blockers and Detergents have already been added to the sample and conjugate pad. 150ul of diluted sample was added per strip.
   The detector are blue latex microspheres conjugated to the detector antibody
   STRIPS LOD: 0,025ng/µL
2) Commercial kit bought as positive testing control (COVID-19 Antigen Rapid Test REF BSD400 cod 600441 Biosigma s.r.l.).
   KIT LOD: 0,0029ng/µL
The following antigens were tested with the Millipore Antibody pair Strips and with the Biosigma Kit (above):
- 2019-nCoV Nucleocapsid Recombinant Antigen, full length (Ag-4) | AGX804
- 2019-nCoV Nucleocapsid Recombinant Antigen, full length (Ag-6) | AGX806
- 2019-nCoV Nucleocapsid Recombinant Antigen, full length (Ag-8) | AGX808
The study has given positive testing results: The capillary transfer from the 3cm FFP2 FILTER to the SAMPLE PAD showed detection even at low antigen concentration value.

## Claims

1. A rapid test method for determining exposure of a mammal to an infectious agent, the method comprising;
a. collecting a sample from the mammal using a wearable device comprising a sample collection element, and
b. determining the presence of the infectious agent or infectious agent materials in the sample collection element,
wherein the sample collection element is collecting a sample from exhaled breath of the mammal and wherein the presence of the infectious agent or infections agent materials in the sample collection element indicates exposure of the mammal to the infectious agent.

2. The method of claim 1, wherein after step a) and prior to step b) the sample collection element is removed from the wearable device.

3. The method of any preceding claim, wherein the infectious agent is an airborne infectious agent.

4. The method of claim 3, wherein the airborne infectious agent is a corona virus or an influenza virus

5. The method of claim 4, wherein the coronavirus is SARS-CoV-2.

6. The method of any one of the preceding claims, wherein the wearable device is a protective gear.

7. The method of claim 6, wherein the protective gear is a face mask.

8. The method of any one of the preceding claims, wherein the sample collection element comprises a testing element.

9. The method of claim 8, wherein the testing element comprises a rapid antigen test for the infectious agent.

10. The method of claim 9, wherein the rapid antigen test is selected from a lateral flow immuno- assay, an aptamer-based test, and a combination thereof.

11. The method of any one of the preceding claims, wherein the sample collection element is worn by the mammal for a predetermined amount of time.

12. The method of claim 11, wherein the predetermined amount of time is at least 30 min.

13. A protective gear against an infectious agent comprising a sample collection element.

14. The protective gear of claim 13, wherein the protective gear is a face mask.

15. The protective gear of claim 13 or 14, wherein the sample collection element comprises a testing element.

16. The protective gear of claim 15, wherein the testing element comprises a rapid antigen test for the infectious agent.

17. The protective gear of any one of claims 13 to 16, wherein the infectious agent is an airborne infectious agent.

18. The protective gear of claim 17, wherein the airborne agent is SARS-CoV-2.
